# EUROPEAN PATENT APPLICATION

(11) **EP 3 967 748 A1**
(43) Date of publication of application: **16.03.2022**
(21) Application number: 20802147.7
(22) Date of filing: 05.05.2020
(51) Int. Cl.: C12N 5/07, C12N 5/075, C12P 19/04, A61L 27/20, A61L 27/60, C08L 1/02, C12R 1/02

(54) **NANOCELLULOSE 3D MATRIX FOR CULTIVATING HUMAN AND ANIMAL CELLS IN VITRO**

(30) Priority: 06.05.2019 BR 102019009242
(71) Applicant: Biocelltis Biotechnologia S/A, 88032-005 Florianópolis (BR)
(72) Inventor: KOEPP, Janice, 88053-446 Florianópolis (BR); BERTI, Fernanda Vieira, 88865-000 Nova Veneza (BR); COLLA, Guilherme, 88310-160 Itajaí (BR); DOS REIS, Emily Marques, 92032-700 Canoas (BR); RAMBO, Carlos Renato, 88037-416 Florianópolis (BR); PORTO, Luismar Marques, 88034-605 Florianópolis SC (BR)
(74) Representative: Pereira Garcia, João Luís
(86) International application number: PCT/BR2020/050151
(87) International publication number: WO 2020/223778

(57) **Abstract**

Standardized nanocellulose 3D matrices for in vitro human and animal cell culture with batch-to-batch regularity in terms of porosity on both surfaces, measured in percent, as well as elasticity, measured by Young's Modulus. A method of manufacturing these 3D nanocellulose matrices, in addition to the matrices manufactured by this method, modified for obtaining nanocellulose 3D matrices containing distinct distribution of nanofibers on the matrix surfaces, considering or not the immobilization, absorption or adsorption of other chemical molecules, resulting in bioengineered physical, chemical, biological and mechanical properties for obtaining an in vitro platform to be used in the cultivation of human and animal cells where the behavior of these cells is evaluated on a time scale (4D). The present invention further encompasses the use of these bioengineered nanocellulose 3D matrices in the development of reconstructed artificial skin in the laboratory with the intention of serving as a platform for testing the efficacy and safety of cosmetics and drugs in vitro, as a platform for in vitro culture of animal and human cells, as a 3D platform for in vitro cytotoxicity and genotoxicity testing, as a platform for in vitro ferti l ization.

## Description

### FIELD OF THE INVENTION

The present invention relates to novel nanocellulose 3D matrices as to their standardization and batch-to-batch regularity in terms of porosity on both surfaces, measured in percentage, as well as their elasticity, measured by Young's Modulus. It also relates to the manufacturing method of these 3D nanocellulose matrices, which was developed to obtain nanocellulose 3D matrices containing distinct distribution of nanofibers on the matrix surfaces, considering or not the immobilization or adsorption, or absorption of other molecules with chemical, biochemical and/or biological activity. It also encompasses the use of these bioengineered nanocellulose 3D matrices in the development of laboratory reconstructed artificial skin with the intention of serving as a platform for testing the efficacy and safety of cosmetics and drugs in vitro, as a platform for animal and human cell culture in vitro, as a 3D platform for cytotoxicity and genotoxicity testing in vitro, as a platform for fertilization in vitro.

### PRIOR ART

Advances in modern medicine depend directly on the quality of results obtained from studies using various experimental methods to analyze the molecular mechanisms involved in the progression of a given disease. Assays performed in vitro combined with results obtained in experimental models in vivo are used to define the treatment of various diseases, understand the molecular mechanisms that trigger diseases, evaluate drug efficacy, as well as assist in the development of increasingly efficient drugs and personalized treatments.

Recently, limitations regarding the use of animals as in vivo experimental models have encouraged the development of increasingly complex and realistic in vitro experimental models. The complexity of these in vitro models refers to the development of new models or experimental platforms that accurately mimic, in the laboratory, the functional dynamics of a tissue, organ or even a developing organism. Currently, culture plates (2D) have been used as in vitro experimental platforms, where human and animal cells grow on the 2D polymeric surface for evaluation of cytotoxicity and drug efficacy, for example.

But unfortunately, the 2D option is very limited and inadequate. Recently, tissue engineering (tissue engineering) approaches have emerged in order to prove that the 2D human and animal cell culture system is unrealistic and does not mimic the behavior of cells in tissues and organs that are three-dimensional (3D) structures. In this context, the development of complex biomaterials capable of mimicry in vitro the natural microstructural complexity of tissues and organs in vivo becomes necessary.

Tissue Engineering has proven that the microenvironment (3D microstructure) where animal cells are cultured is essential for them to be able to express the same genes expressed in vivo. After all, human tissues and organs are arranged in a 3D microenvironment when nanometrically analyzed. These recent findings prove that the development and testing of drugs and cosmetics for cytotoxicity, efficacy and safety should no longer be performed on 2D experimental platforms, but on 3D experimental platforms.

### 2D animal cell culture

Experimental platforms for in vitro animal cell culture have been used since the early 1900s in 2D culture plates. 2D animal cell culture refers to the growth of these cells on culture plates typically made of plastic material (polymer of petrochemical origin). The animal cells are placed on the surface of these plastic plates that have a specific surface treatment for the cells to adhere and proliferate. Unfortunately, these 2D environments do not represent the microstructure of organs and tissues in vivo, which causes the cells to change their behavior to adapt to the 2D condition. Thus, 2D cell culture is not a representative platform for the natural biological microenvironment of tissues and organs. Consequently, cell culture on a 2D platform is not a suitable experimental model to understand how cells grow and their function in the human body where they are surrounded by other cells and extracellular matrix (ECM) in three dimensions. Tests with 2D cell culture are not always predictive, which increases the cost and failure rate in new drug discovery and clinical trials performed by the pharmaceutical and cosmetic industry.

### 3D animal cell culture

Recently, the development of biomaterials as 3D platforms for animal cell culture has been a focus of research and development (R&D) in the area of Tissue Engineering. The main goal in this segment is to develop realistic, dynamic and personalized tissue models in vitro.

An ideal 3D experimental platform should provide a suitable microenvironment for cell adhesion, proliferation, differentiation, migration and invasion (PEPPAS, N. A. et al. Hydrogels in biology and medicine: From molecular principles to bionanotechnology. Advanced Materials, v. 18, n. 11, p. 1345-1360, 2006). Alternatively, hydrogels have been used as platforms to enable the growth of healthy and diseased animal cells by combining conformational signaling molecules in the 3D microenvironment. In this context, hydrogels provide dynamic microenvironments that resemble the extracellular matrix (ECM) and determine cell fate through cell-cell or cell-hydrogel interactions (EL-SHERBINY, I. M.; YACOUB, M. H. Hydrogel scaffolds for tissue engineering: Progress and challenges. Global Cardiology Science and Practice, v. 2013, n. 3, p. 38, 2013).

The creation of in vitro 3D tissue platforms has enabled the advancement of regenerative medicine in the last decade. These platforms have allowed the most sophisticated 3D stem cell culture to be transformed into organ-like structures. Slowly, 2D cell culture is becoming extinct, yet creating a 3D platform is not enough to mimic in vitro the dynamism of tissues and organs in vivo. The need to add another dimension to 3D cell culture is already a reality. This new dimension allows cells seeded on 3D platforms to self-organize autonomously and for this, analyses need to be performed on a time scale, i.e., a fourth dimension of the in vitro bioengineered tissue (4D).

One of the recent trends is incorporation of peptides, DNA, oligonucleotides and nanoparticles to create "programmable hydrogels" which allows the construction of a programmable microenvironment for each tissue (KE, Y.; ONG, L. L.; SHIH, W. M.; YIN, P. Three-Dimensional Structures Self-Assembled from DNA Bricks. Science, 30 Nov 2012:Vol. 338, Issue 6111, pp. 1177-1183.DOI: 10.1126/science. 1227268).

The native ECM is a highly complex fibrous matrix, composed of proteins (e.g. collagen, laminin, fibronectin, elastin), glycosaminoglycans (e.g. hyaluronic acid, heparin), proteoglycans (e.g. perlecan, syndecan), and growth factors that play an important role in cell behavior, interfering in the processes of differentiation, proliferation, invasion and apoptosis (KULAR, J. K.; BASU, S.; SHARMA, R. I. The extracellular matrix: structure, composition, age-related differences, tools for analysis and applications for tissue engineering. Journal of tissue engineering, v. 5, p. 1-17, 2014). The ECM acts not only as a mechanical support structure for cells, but also as a bioactive dynamic microenvironment that mediates cellular functions (RHODES, J. M.; SIMONS, M. The extracellular matrix and blood vessel formation: not just a scaffold. Journal of cellular and molecular medicine, v. 11, n. 2, p. 176-205, 2007). Thus, the incorporation of bioactive molecules into hydrogels is an important tool to manufacture platforms that mimic in vitro the functional microenvironment of cells in vivo (ZHU, J.; MARCHANT, R. E. Design properties of hydrogel tissue-engineering scaffolds. Expert review of medical devices, v. 8, n. 5, p. 607-26, 2011).

Nanocellulose is a hydrogel secreted by some strains of bacteria such as those of the genus *Gluconacetobater, Agrobacterium, Pseudomonas, Rhizobium,* and *Sarcina* under specific bacterial culture conditions. The nanocellulose is secreted by the bacteria in the form of hydrophilic nanofibers that resemble the native ECM (RAMBO, C. R. et al. Template assisted synthesis of porous nanofibrous cellulose membranes for tissue engineering. Materials Science and Engineering C, v. 28, n. 4, p. 549-554, 2008). Chemically, nanocellulose is composed of glucose monomers linked together by β(1-4) glycosidic bonds of chemical formula (C6H1005)n (PARK, J.; PARK, Y.; JUNG, J. Production of bacterial cellulose by Gluconacetobacter hansenii PJK isolated from rotten apple. Biotechnology and Bioprocess Engineering, v. 8, p. 83-88, 2003). More specifically, it is a cellobiose polymer, which is unit repeating polymerization. The microstructure of the nanocellulose hydrogel gives it unique properties such as water holding capacity, mechanical strength, porosity and biocompatibility (KLEMM, D. et al. Bacterial synthesized cellulose - Artificial blood vessels for microsurgery. Progress in Polymer Science (Oxford), v. 26, n. 9, p. 1561-1603, 2001). Therefore, we call the artificially created microenvironment in which animal and human cells can grow and interact with the microenvironment in all three dimensions a 3D matrix. Unlike 2D environments (e.g. a Petri dish), 3D cell culture allows cells to grow/proliferate/migrate in vitro in all directions, just as they do in vivo.

### Biocompatibility

The biocompatibility of nanocellulose has been proven by Helenius et al. (HELENIUS, G., H. BACKDAHL, A. BODIN, U. NANNMARK, P. GATENHOLM and B. RISBERG. In vivo biocompatibility of bacterial cellulose. J Biomed Mater Res A, v.76, n.2, p.431-8. 2006) in an in vivo study. In this study, it was shown that nanocellulose has the ability to integrate into the human tissue to be treated without causing inflammatory reaction. Because it is a tough, biocompatible, inert biomaterial, nanocellulose is considered an ideal hydrogel for creating 3D platforms for in vitro cell culture. The fact that nanocellulose is an inert hydrogel and is not biodegradable when in contact with animal and human cells makes it one of the potential biomaterials for the development of 3D platforms, as no spontaneous release of degradation product occurs in the cell culture medium.

Nanocellulose has been studied as a platform for 3D cell culture by several research groups in recent years. Even though it is considered as a widely studied biomaterial for a variety of applications, the microstructural and mechanical properties of nanocellulose have been little explored over these years. The potential use of nanocellulose in endothelial cell culture (BERTI, F. V. et al. Nanofiber density determines endothelial cell behavior on hydrogel matrix. Materials Science and Engineering C, v. 33, n. 8, p. 4684-4691, 2013.), fibroblasts, stem cells (DE OLIVEIRA, C. R., CARVALHO, J. L., NOVIKOFF, S., BERTI, F. V.; PORTO, L. M., GOMES, D., GOES, A. M. Bacterial Cellulose Membranes Constitute Biocompatible Biomaterials for Mesenchymal and Induced Pluripotent Stem Cell Culture and Tissue Engineering. de Oliveira et al. J Tissue Sci Eng 2012, S:11. http://dx.doi.org/10.4172/2157-7552.S11-005) and melanoma cells (DOS REIS, E. M., BERTI, F. V., COLLA, G., PORTO, L. M. Bacterial nanocellulose-IKVAV hydrogel matrix modulates melanoma tumor cell adhesion and proliferation and induces vasculogenic mimicry in vitro. J Biomed Mater Res Part B, 2018 Nov, v.106 (8): 2741-2749. DOI: 10.1002/jbm.b.34055) in vitro were evaluated and confirmed that the material is not cytotoxic to human and animal cells. The importance of the microstructural properties of nanocellulose in directing the behavior of human cells was reported by Berti (BERTI, F. V. et al. Nanofiber density determines endothelial cell behavior on hydrogel matrix. Materials Science and Engineering C, v. 33, n. 8, p. 4684-4691, 2013) only in 2013. This study proved that the same cells, in this case human endothelial cells, showed different behaviors when grown on the surface of nanocellulose containing different porosities. Even after scientific evidence proves how important the microstructural properties of nanocellulose are in determining cell fate, there are many recent studies that do not take this and other important factors into consideration. Even existing patents to date do not indicate the microstructure of nanocellulose as an important property for cell fate determination in vitro, nor even the use of 3D nanocellulose in the temporal analysis of animal and human cell behavior.

Other platforms available for 3D cell culture in vitro such as Geltrex^{®} and Matrigel^{®} products are produced from extracellular matrix (ECM) extracted from animals in vivo. These platforms have a great variability regarding physical, chemical and mechanical properties when comparing each batch of products. This variability present in the commercial products cited above confirms the need for development of standardized and optimized biomaterials that mimic the ECM in vitro.

The physical, chemical and mechanical properties of biomaterials determine the biological responses of cells when they are cultured in vitro. Researches in Tissue Engineering and Regenerative Medicine have proven that the control of physical, chemical and mechanical properties of biomaterials is fundamental to direct cell behavior in vitro and consequently to mimic several cell communication mechanisms that confer functionality to the most diverse tissues and organs in vivo.

Thus, the creation of biomaterials containing previously bioengineered physical, chemical and mechanical properties is essential for representing or mimicry functional cellular mechanisms of tissues and organs in vitro. In addition to culturing human and animal cells in bioengineered 3D microenvironments one should take into consideration an additional parameter: the in vitro cultivation time.

The temporal analysis of biological phenomena that occur in 3D microenvironments in vitro is fundamental to mimic the several simultaneous reactions that happen in the cells until they organize themselves to acquire a specific tissue function. These temporal analyses of biological phenomena in vitro are no longer called 3D but 4D, where time is added as an additional parameter of analysis.

In this context, nanocellulose is considered an extremely interesting biomaterial because it is an inert, biocompatible polymer that can be bioengineered and has the ability to retain 99% of its volume in water, as well as elements of the extracellular matrix secreted by cells, and that provide mechanical and structural support to human and animal tissues and organs.

Nanocellulose can be produced in static bacterial culture from which a structured matrix is obtained composed of an ultrafine three-dimensional network of cellulose nanofibers capable of retaining about 99% of water (KLEMM, D. et al. Bacterial synthesized cellulose - Artificial blood vessels for microsurgery. Progress in Polymer Science (Oxford), v. 26, n. 9, p. 1561-1603, 2001, KLEMM, D. et al. Nanocelluloses: A new family of nature-based materials. Angewandte Chemie - International Edition, v. 50, n. 24, p. 5438-54662011, 2011). When nanocellulose is produced under static fermentation conditions, generally two distinct surfaces are formed, the top and bottom surface of the matrix. The upper surface is understood as the side in contact with the gas phase of the medium in static culture.

The distribution of the nanocellulose nanofibers on the top and bottom surfaces directly depends on the culture medium used in bacterial fermentation, the bacterial strain used, the culture time, the oxygenation of the fermentation medium, and the use or not of molds to obtain pores in the matrix.

During fermentation, the side of the matrix that forms at the air (or 02-containing) / liquid interface has a higher density of nanofibers (top surface) while the opposite side shows a lower density of nanofibers and a more porous surface (bottom surface) (BERTI, F. V. et al. Nanofiber density determines endothelial cell behavior on hydrogel matrix. Materials Science and Engineering C, v. 33, n. 8, p. 4684-4691, 2013). Recently it was found that differences regarding the density of nanofibers on the top and bottom surface of the matrix can be modulated through the composition of the culture medium in terms of carbon and nitrogen source (DE SOUZA, S. S., CESCA, K., SCHROEDER, C., NASCIMENTO, F. X., BERTI, F. V., PORTO, L. M. Optically transparent bacterial nanocellulose scaffolds for tissue engineering. Proceedings of the Brazilian Congress of Chemical Engineering, COBEQ 2016. Accessed at: https://proceedings.science/proceedings/44/_papers/40064/download/fulltext_file3 on 06 May 2019. and DE SOUZA, S. S., BERTI, F. V., DE OLIVEIRA, K. P. V., PITELLA, C. Q., DE CASTRO, J. V., PELISSARI, C., RAMBO, C. R., PORTO, L. M. Nanocellulose biosynthesis by Komagataeibacter hansenii in a defined minimal culture medium. Cellulose (2019) 26: 1641. https://doi.org/10.1007/s10570-018-2178-4). These findings further expand the potential for using nanocellulose matrices in tissue engineering applications.

Currently, various animal-based assays are used to evaluate immunotoxic effects, such as immunosuppression and sensitization. The use of animals, however, presents several problems, including economic and ethical, and relevance to human risk assessment. It is increasingly believed that non-animal approaches can eliminate these problems without jeopardizing human safety.

According to the Brazilian Association of Personal Care, Perfumery and Cosmetic Industries (ABIHPEC), Brazil is the world's fourth largest market for beauty products, behind only the United States, China, and Japan. A market that moved approximately R$ 13.81 billion (2017), where the main sales categories are perfumery and moisturizing creams (ABIHPEC, 2017). As of September 2019, in a decision by the National Council for the Control of Animal Experimentation (CONCEA) it was determined that Brazil will not be allowed to test cosmetics on animals. Thus, CONCEA listed 17 alternative methods to the use of animals in testing, validated by the Organization for Economic Co-operation and Development (OECD).

**THE 17 ALTERNATIVE METHODS RECOGNIZED BY CONCEA**

| Potential for skin irritation and corrosion | |
|---|---|
| 1) OECD TG 430 | In vitro dermal corrosion: transcutaneous electrical resistance test |
| 2) OECD TG 431 | In vitro dermal corrosion: reconstituted human epidermis test |
| 3) OECD TG 435 | In vitro membrane barrier test |
| 4) OECD TG 439 | In vitro skin irritation test |
| Potential for eye irritation and corrosion | |
| 5) OECD TG 437 | Bovine corneal permeability and opacity test |
| 6) OECD TG 438 | Isolated chicken eye test |
| 7) OECD TG 460 | Fluorescein permeation test |

| Phototoxicity potential | |
|---|---|
| 8) OECD TG 432 | In vitro phototoxicity test 3T3 NRU |

| Cutaneous Absorption | |
|---|---|
| | |
| 9) OECD TG 428 | In vitro cutaneous absorption method |

| Skin sensitization potential | |
|---|---|
| 10) OECD TG 429 | Skin sensitization: local lymph node assay |
| 11) OECD TG 442A | Non-radioactive version of the local lymph node assay |
| 12) OECD TG 442B | Non-radioactive version of the local lymph node assay |

| Acute Toxicity | |
|---|---|
| 13) OECD TG 420 | Acute oral toxicity: fixed dose procedure |
| 14) OECD TG 423 | Acute oral toxicity: acute toxic class |
| 15) OECD TG 425 | Acute oral toxicity: "up and down" procedure |
| 16) OECD TG 129 | Estimation of initial dose for systemic acute oral toxicity testing |

| Genotoxicity | |
|---|---|
| 17) OECD TG 487 | In vitro mammalian cell micronucleus test |

Of the 17 alternative methods, two require an equivalent human epidermis to be used in the validation of cosmetics (CONCEA, 2015). One of them serves to ascertain the irritation potential of new products and the other serves for the evaluation of corrosion of the tested substances. However, these validated models are only produced abroad and are difficult to access in Brazil due to logistical and customs issues. The objective of performing these tests is to know if the product in question has a corrosive or irritating action before reaching the patient. Several substances have this attribute of being a corrosive agent that when they come into contact with the skin, degenerate it forming a wound with dead cells, and the tissue goes into a process of necrosis.

The development and validation of an artificial skin that simulates human skin is an area of comprehensive potential, which can leverage the end of the use of animals for cosmetic safety testing. In addition, the skin can be used to evaluate a range of dermatological diseases.

Currently, the artificial skin market is led by the multinational L'Oreal, through the company EpiSkin, which, per year, produces around 150,000 reconstructed skin units, and another 30,000 pigmented skin tissues, which are distributed in Europe in kits of 24 units. In addition, L'Oreal commercializes other skin models abroad, simulating various types of epithelium, such as mucous membranes of the mouth, gums and vagina. In the US, the company MatTek markets several equivalent skin models similar to those manufactured by L'Oreal, and the startup OneSkin simulates human skin aging in vitro.

Although the Brazilian legislation allows the import of artificial skin, this option is practically unfeasible, considering that, because it is a living material, the skin fragments are kept in ideal conditions for only a few days. And taking into consideration the frequent customs problems, the delay in arrival of this material would make its use unfeasible. In this scenario, the OECD encourages the production of new RHE models for skin irritation testing as described in detail in its OECD guide No. 439 (OECD, 2015), about the quality control and performance parameters that a model should present. Thus, there is a need for development and validation of an RHE model in order to allow this technology to be available to Brazilian cosmetic industries.

Skin models are currently developed on different types of supports, among them matrices that resemble the extracellular matrix, collagen supports or inert filters. The advantages of using biopolymer platforms as cell support are mainly associated with their structure, which is similar to that of tissue in vivo, and biocompatibility with cells.

What is apparent from the above papers is that obtaining nanocellulose 3D matrices for in vitro human and animal cell culture with defined and known porosities on both surfaces is highly desirable due to their potential use in a wide variety of applications, such as growing in vitro reconstructed human skin from human and animal cells for use in efficacy and safety testing of dermocosmetics, growing animal and human embryonic cells in in vitro fertilization processes, among others. However, obtaining nanocellulose matrices with defined and known porosities on both surfaces is not an easy task, and today the products available on the market show great variability in terms of physical, chemical, and mechanical properties when comparing each batch. In addition, there is still no method described in the state of the art that modulates the obtainment of nanocellulose matrices with distinct distribution of nanofibers on the matrix surfaces.

To solve this problem of the technique, the inventors have developed a novel method, which allows modulation of the porosity of the nanofiber distribution of both surfaces of the matrix by adjusting, in the fermentation step, the culture medium, the nitrogen source, whether complex or non-complex, in addition to the amount of days for fermentation and the exposure of the surfaces during this process.

As the prior art was unaware until 2013 of the importance of the microstructural properties of nanocellulose in directing the behavior of human cells, the use of these bioengineered matrices in terms of the distribution of nanofibers on the matrix surfaces was developed by the inventors of the present invention for the applications of developing reconstructed artificial skin in the laboratory with the intention of serving as a platform for testing the efficacy and safety of cosmetics and drugs in vitro, as a platform for in vitro animal and human cell culture, as a 3D platform for in vitro cytotoxicity and genotoxicity testing, as a platform for in vitro fertilization.

### SUMMARY OF THE INVENTION

The present invention encompasses novel features of 3D nanocellulose matrices, as to their standardization and batch-to-batch regularity in terms of porosity, on both surfaces, measured in percentage, as well as their elasticity, measured by Young's Modulus. It also encompasses the manufacturing method of these 3D nanocellulose matrices, besides the matrices manufactured by this method, through the adjustment, in the fermentation step of the culture medium, of the nitrogen source, whether complex or non-complex, besides the amount of days for fermentation and the exposure of the surfaces during this process, resulting in bioengineered physical, chemical and mechanical properties to obtain an in vitro platform to be used in the cultivation of human and animal cells where the behavior of these cells is evaluated in a time scale (4D). The fermentation method developed in the present invention is modified to obtain nanocellulose 3D matrices containing distinct distribution of nanofibers on the matrix surfaces, considering or not the immobilization, adsorption or absorption of other chemical molecules.

The present invention further encompasses the use of these bioengineered nanocellulose 3D matrices in the development of reconstructed artificial skin in the laboratory with the intention of serving as a platform for testing the efficacy and safety of cosmetics and drugs in vitro, as a platform for animal and human cell culture in vitro, as a 3D platform for cytotoxicity and genotoxicity testing in vitro, as a platform for fertilization in vitro.

### DESCRIPTION OF THE DRAWINGS

The drawings illustrate certain aspects of some of the embodiments of the present invention and should not be used to limit or define the invention.

Along with the description, the drawings serve to explain certain principles of the invention.
FIG. 1 is the results of evaluation of nanocellulose microstructure after treatment of micrographs of the 3D matrix obtained by SEM.
FIG. 2 is a scanning electron microscopy image of nanocellulose 3D matrix containing a surface having a dense distribution of nanofibers (0.1 to 10%, left image) and the opposite surface containing a porous distribution of nanofibers (10 to 99%, right image).
FIG. 3 is a scanning electron microscopy image of nanocellulose 3D matrix containing both surfaces (right and left images) with dense nanofiber distribution (0.1 to 10%).
[040] FIG. 4 is a scanning electron microscopy image of nanocellulose 3D matrix containing both surfaces (right and left images) with porous distribution of nanofibers (10 to 99%).
FIG. 5 is a graphic showing the incorporation of melatonin into the nanocellulose 3D matrix for use in in vitro fertilization processes or other steps involved in in vitro embryo production (IVOP).
FIG. 6 is an image of the maturation of oocytes cultured on the porous surface of the 3D nanocellulose matrices.
FIG. 7 is an image of the maturation of oocytes cultured on the dense surface of the 3D nanocellulose arrays.
FIG. 8 is an image of reconstructed human skin over the nanocellulose 3D matrix containing a porous distribution of nanofibers on the surface of the matrix. The image is an enlargement of the self-organization of human fibroblasts, components of the human dermis that have adhered, proliferated and self-organized over the nanofibers of the nanocellulose 3D matrix.
FIG. 9 is a scanning electron microscopy image of human fibroblasts grown on the nanocellulose 3D matrix on the surface with dense distribution of nanofibers (0.1 to 10%).

### DETAILED DESCRIPTION OF THE INVENTION

The present invention encompasses novel characteristics of 3D nanocellulose matrices, as to their standardization and batch-to-batch regularity in terms of porosity, on both surfaces, measured in percentage, as well as their elasticity, measured by Young's Modulus.

The physical properties referred to in the invention provide for obtaining nanocellulose matrices containing one of the surfaces with a dense distribution of nanofibers and the other surface with a light distribution of nanofibers, therefore more porous than the previous one; or yet, the production of a nanocellulose matrix containing both porous surfaces; or yet both surfaces with a dense distribution of cellulose nanofibers, therefore a non-porous matrix.

Another relevant physical property is the thickness of the nanocellulose matrix that can be controlled by the fermentation time under static bacterial culture conditions from which a 3D matrix is obtained.

The distribution and density of nanofibers on the surfaces of the nanocellulose matrix significantly change the mass and volume of the produced matrix, so they are relevant parameters when obtaining bioengineered hydrogels. The density and structural organization of nanofibers interferes directly in parameters related to crystallinity and transparency, among other physical properties.

There are several protocols available in the literature aimed at characterizing the microstructure of biomaterials. The biggest challenge in this case is related to the fact that the mode of sample preparation is directly dependent on the type of biomaterial. Thus, there are no standard protocols previously defined.

A method that can be used for determining microstructural properties of hydrogels is the BET Method or even the Multimolecular Adsorption Theory which is a mathematical theory aiming to describe the physical adsorption of gas molecules on a solid surface, and which serves as the basis for an important analysis technique for measuring the specific surface area of a material; from these data obtained, mathematically it is possible to determine the porosity and nanofiber density of the nanocellulose 3D matrix when dehydrated and dried.

The method we use in the present invention to characterize the microstructure of nanocellulose 3D matrices and thus define the parameters that identify these matrices, is a method previously optimized by our inventors, which allows the evaluation of the nanocellulose microstructure in terms of porosity, pore diameter, nanofiber diameter among other properties related to the microstructure (BERTI, F. V. et al. Nanofiber density determines endothelial cell behavior on hydrogel matrix. Materials Science and Engineering C, v. 33, n. 8, p. 4684-4691, 2013). The preparation of nanocellulose samples for microstructure analysis involves the use of scanning electron microscopy (SEM). Then the nanocellulose hydrogel samples are dehydrated with serial ethanol solutions (20%, 30%, 50%, 70%, 80%, 90% and finally 100%) considering repetitive execution of each dehydration step until total water exclusion. The nanocellulose samples are subsequently placed in equipment called Critical Point Drying (CPD). CPD dehydration is an established method of dehydrating biological materials and tissues that precedes their analysis by SEM. The Critical Point Drying (CPD) technique was first introduced commercially for sample preparation by Polaron Ltd in 1971. The concepts of characterization and preparation of hydrated biological samples have been proposed as an alternative for observation by scanning electron microscopy (SEM), with the main objective to be structurally and morphologically characterized (BRAET, F., R. DE ZANGER and E. WISSE. Drying cells for SEM, AFM and TEM by hexamethyldisilazane: a study on hepatic endothelial cells. J Microsc, v.186, p. 84-7. 1997; ECHLIN, P. Handbook of sample preparation for scanning electron microscopy and x-ray microanalysis. Boston: Springer. 2009). After dehydration and drying by CPD the nanocellulose is prepared in the conventional way for microstructure analysis by SEM. The micrographs obtained by SEM are then analyzed by software optimized for determining porosity, pore diameter and diameter of nanofibers, such as the Image J software. From the analysis of the microstructures in the image J software the porosity of the analyzed area is obtained as a percentage.

In our case, the study to evaluate the microstructure of the nanocellulose in terms of porosity, pore diameter, diameter of nanofibers among other properties related to the microstructure found was as follows:

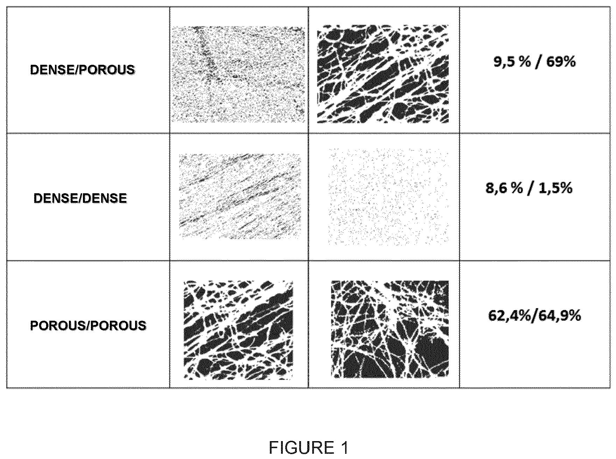

After treatment of the micrographs of the 3D matrix obtained by SEM the results shown in FIG. 1 were obtained, and the porosity values can be bioengineered according to the need of the intended application for the matrix.

The nanocellulose 3D matrices have diameter variations between 0.1 mm and 600 mm and thickness variations between 0.1 mm and 200 mm and can be produced in other formats besides cylindrical aiming at the same applications required in this invention.

Transparency is a relevant property because it facilitates the monitoring of biological phenomena in microscopic observations that require the passage of light through the membrane. Transparency, as well as color, can be altered according to the composition of the culture medium used in the bacterial fermentation process or the insertion of other composite materials (resins for example) that confer transparency to the nanocellulose matrix.

The mechanical properties of the nanocellulose matrix depend on the modifications in physical and chemical properties performed previously on the nanocellulose matrices. Elasticity, mechanical tensile strength, elongation at break, among others, are examples of properties that can be modulated according to the production method of the nanocellulose matrices.

The elasticity of nanocellulose 3D matrices is an important parameter because it allows the handling of in vitro models when there is a need to transfer the in vitro tissue between one equipment and another to evaluate the biological and physicochemical characteristics that would be limited if the biomaterial did not have compatible strength. The mechanical properties of the nanocellulose 3D matrices were measured in a texturometer using the samples without cuts and in the hydrated condition.

The mechanical properties of nanocellulose 3D matrices can be changed according to their production method, but as a reference we measured some parameters of nanocellulose 3D matrices produced in seven days of fermentation containing the following dense/porous and porous/porous configurations, in this case the following results were obtained:

| | Porous/Porous | Dense/Porous |
|---|---|---|
| Elongation (%) | 50.93 | 99.98 |
| Tensile Strength (MPa) | 0.521 | 0.357 |
| Tenacity (J/m3) | 57.8 | 46.6 |

The biological properties of the nanocellulose matrix are defined through in vitro testing where animal and human cells are cultured on the matrix in order to evaluate various mechanisms related to cell adhesion, proliferation, migration, and differentiation. Specifically, the nanocellulose matrix serves as a 3D platform capable of supporting adhesion, proliferation, migration and differentiation of animal and human cells where, consequently, one can control and evaluate the dynamics of cell behavior in 3D microenvironments on a 4D time scale.

The present invention encompasses the method of manufacturing 3D nanocellulose arrays, in addition to the arrays manufactured by this method, by adjusting, in the fermentation step of the culture medium, the nitrogen source, whether complex or non-complex, in addition to the amount of days for fermentation and the exposure of the surfaces during this process, resulting in bioengineered physical, chemical and mechanical properties to obtain an in vitro platform that is used in the cultivation of human and animal cells where the behavior of these cells is evaluated on a temporal scale (4D). The fermentation method developed in the present invention is modified to obtain nanocellulose 3D matrices containing distinct distribution of nanofibers on the surfaces of the matrices, considering or not the immobilization or adsorption of other chemical molecules.

The present invention relates to a novel method of manufacturing nanocellulose 3D matrices bioengineered as to their physical, chemical, biological and mechanical properties that are used as a platform for temporal (4D) culture of human and animal cells in order to reproduce in vitro the self-organization of these cells and the mimicry of biological mechanisms involved in tissue functionalization in vivo.

Bioengineered nanocellulose matrix means the changes in physical, chemical and mechanical properties that can be performed through changes in the manufacturing method of the nanocellulose 3D matrix in order to meet the requirements necessary for the induction of biological responses in vitro that mimic functional mechanisms present in tissues in vivo.

After several process optimizations it was found that the fermentation medium used to grow the bacteria directly influences the physical and mechanical properties of the obtained nanocellulose matrix. The amount and type of carbon sources, nitrogen and other components used in the fermentation of the bacteria *Gluconacetobacter hansenii, Gluconacetobacter xylinus, Komagataeibacter hansenii* or even *Acetobacter hansenii* alter the mechanism of secretion of cellulose nanofibers by the bacteria. Thus, a nanocellulose matrix containing specific physical and mechanical properties can be bioengineered by modulating the composition of the culture medium.

Besides the constitution of the culture medium other parameters are relevant and cause changes in the physical and mechanical properties of the nanocellulose matrix under fermentation. The bacterial strain used in the process, the concentration of bacteria in the medium, the chemical constitution of the medium, the fermentation time and temperature, oxygenation, the use of artifacts as structural barriers, the pH of the medium, and the way the fermentation process is conducted, which can occur in a static or dynamic manner.

Changes in the chemical properties of the nanocellulose matrix are mainly accomplished through the incorporation or immobilization of molecules inducing and signaling biological responses. The modification of the chemical properties occurs directly on the free hydroxyl groups present in the nanocellulose matrix.

The present invention provides for the incorporation of biological response signaling molecules, which occurs by immersing the nanocellulose matrices in an aqueous or non-aqueous solution containing the molecules of interest. After incorporation of the bioactive molecules the same can be released in order to induce or signal biological responses in animal and human cells grown in the nanocellulose matrix over culture time.

The present invention provides for adsorption and/or uptake and immobilization of signaling molecules on the nanocellulose matrix by non-covalent surface modifications, sulfonation, oxidation, esterification, silylation, urethanation, amidation, immobilization by "click chemistry" and polymeric inclusion or "polymer grafting".

The nanocellulose matrix without modification of properties is an inert platform that allows the adhesion and proliferation of animal and human cells that secrete on the nanofibers their own extracellular matrix that serves as molecular signaling for the cells grown there.

The bioengineered nanocellulose matrix can be used as a platform for 3D or 4D culture of animal and human cells in vitro, efficacy and safety testing of drugs and cosmetics, support for in vitro oocyte maturation and embryo development in in vitro fertilization processes.

The bioengineered nanocellulose matrix is obtained through the fermentation process of the bacteria *Gluconacetobacter hansenii, Gluconacetobacter xylinus, Komagataeibacter hansenii* or also *Acetobacter hansenii* among other bacteria of the same species.

The present invention provides the method for manufacturing the 3D nanocellulose matrices, it follows the same method of activation of the bacterial strain provided in patent BR 10 2019 007462 0. However, the fermentation method is changed to obtain nanocellulose 3D matrices containing distinct distribution of nanofibers on the matrix surfaces, considering or not the immobilization, adsorption or absorption of other chemical molecules.

The present invention provides that the fermentation method determines the physical properties of the 3D nanocellulose matrices. To obtain nanocellulose 3D matrices containing:

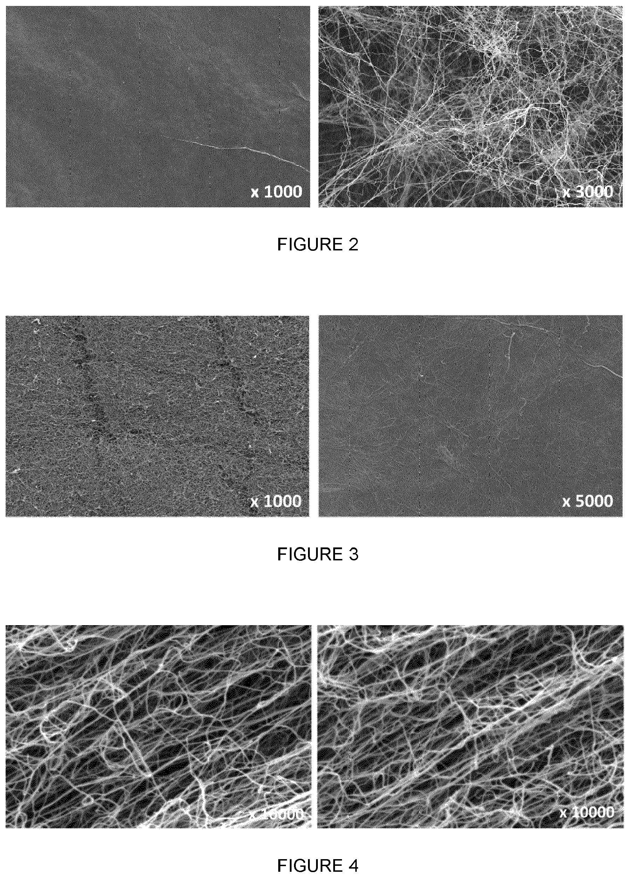

One surface of the matrix with a dense distribution of nanofibers and the other surface with a porous distribution of nanofibers. In this case, the fermentation medium contains one or more carbon sources (glycerol, glucose, fructose, mannitol, sucrose, sorbitol, and galactose) and one or more complex nitrogen sources (peptone and yeast extract). Fermentation is conducted at temperatures between 25 to 30°C, preferably 25 to 26°C for 1 to 10 days, preferably 7 days or less. The use of a complex nitrogen source is essential to obtain a nanocellulose 3D matrix containing distinct nanofiber distribution. After fermentation the nanocellulose 3D matrices are further purified and sterilized. Through this method nanocellulose 3D matrices containing on the dense face porosity from 0.1 to 10% and on the porous face from 10 to 99% and Young's modulus from 0.05 to 60 MPa, preferably 0.05 to 10 MPa are obtained. For the characterization of the distribution of nanofibers on the upper and lower surfaces of the nanocellulose 3D matrices manufactured by this process, these were evaluated by scanning electron microscopy and the results of the microstructural configurations can be seen in FIG. 2, where in the left image we can see a surface with dense distribution of nanofibers, and in the right image we can see on the opposite surface a porous distribution of nanofibers.

Both surfaces of the matrix containing a dense distribution of nanocellulose nanofibers. In this case the fermentation medium contains one or more carbon sources (glycerol, glucose, fructose, mannitol, sucrose, sorbitol and galactose) and one or more complex nitrogen sources (peptone and yeast extract). Fermentation is conducted at temperatures between 25 and 30°C, preferably 25 to 26°C, and after 3 to 5 days of fermentation the nanocellulose matrix is turned over and the opposite surface is exposed to the media/air interface, which is maintained for another 3 to 5 days in fermentation. The use of a complex nitrogen source is essential to obtain a nanocellulose 3D matrix containing the faces with dense distribution of nanofibers. After fermentation, the nanocellulose 3D matrices are further purified and sterilized. Through this method one obtains nanocellulose 3D matrices containing on both sides porosity from 0.1 to 10% and Young's modulus from 0.1 to 4,800 MPa, preferably from 0.1 to 50 MPa. To characterize the distribution of nanofibers on the top and bottom surfaces of the nanocellulose 3D matrices manufactured by this process, these were evaluated by scanning electron microscopy and the results of the microstructural configurations can be seen in FIG. 3, where in the left image we can see a surface with dense distribution of nanofibers and in the right image we can also see on the opposite surface a dense distribution of nanofibers.

Both surfaces of the matrix containing a porous distribution of nanocellulose nanofibers. In this case the fermentation medium contains one or more carbon sources (glycerol, glucose, fructose, mannitol, sucrose, sorbitol and galactose) and one or more non-complex nitrogen sources [selected preferably from ammonium glutamate, ammonium nitrate (NH₄NO₃), ammonium chloride (NH₄CI) and ammonium sulfate ((NH₄)₂SO₄]. Fermentation is conducted at temperatures between 25 and 30°C, preferably 25 to 26°C, which is maintained for 3 to 10 days in fermentation. The absence of a complex nitrogen source is essential to obtain a nanocellulose 3D matrix containing both sides with porous distribution of nanofibers. After fermentation the nanocellulose 3D matrices are further purified and sterilized. Through this method one obtains nanocellulose 3D matrices containing on both sides porosity from 10 to 99% and Young's modulus 0.01 to 0.1 MPa, preferably 0.01 to 0.076 MPa. For the characterization of the distribution of nanofibers on the top and bottom surfaces of the nanocellulose 3D matrices fabricated by this process, these were evaluated by scanning electron microscopy and the results of the microstructural configurations can be seen in FIG. 4, where in the left image we can see a surface with porous distribution of nanofibers and in the right image we can also see on the opposite surface a porous distribution of nanofibers.

Nanocellulose 3D matrices can be chemically modified after the sterilization process or can be used without chemical modification.

The methods used for absorption of bioactive molecules follows what was previously described by the same inventors in the invention BR 10 2019 007462 0.

The methods used for adsorption, absorption and/or chemical immobilization of bioactive molecules can preferably occur through non-covalent surface modifications, sulfonation, oxidation, esterification, silylation, urethanation, amidation, immobilization by "click chemistry" and polymer inclusion or "polymer grafting".

The present invention further encompasses the use of these bioengineered nanocellulose 3D matrices in the development of laboratory reconstructed artificial skin with the intention of serving as a platform for testing efficacy and safety of cosmetics and drugs in vitro, as a platform for 3D culture of animal and human cells in vitro, as a 3D platform for cytotoxicity and genotoxicity testing in vitro, as a platform for fertilization in vitro.

More specifically the present invention encompasses the use of in vitro reconstructed human skin on a bioengineered nanocellulose matrix for efficacy testing of dermocosmetics and drugs; production and use of bioengineered nanocellulose matrix for 3D and 4D culture of animal and human cells in vitro; production and use of bioengineered nanocellulose matrix as a platform for in vitro fertilization and embryo development.

The present invention provides for the use of 3D nanocellulose arrays containing both surfaces with dense distribution of nanofibers; both surfaces with porous distribution of nanofibers; one surface dense and one porous as to distribution of nanocellulose nanofibers. These nanocellulose 3D matrices are used for three applications to name:

3D and 4D cell culture, where human or animal cells are grown on the nanocellulose 3D matrix. The cells grown in vitro are maintained in culture over time to evaluate by analytical methods the cell behavior (adhesion, proliferation and migration), cell fate (differentiation, signaling), functionality, phenotype, gene expression among other mechanisms related to proteome, metabolome, genome and transcriptome.

Growth of human and animal cells present in the epidermis, dermis and hypodermis on the nanocellulose 3D matrix in order to mimic in vitro the above-mentioned tissues. The in vitro reconstructed skins will be used as test platforms to evaluate efficacy and safety of drugs and cosmetics.

Platform support for oocyte maturation and embryo development in animal or human in vitro fertilization processes.

There is a multitude of possibilities for developing 3D tissue models in vitro. These models can mimic from this a microenvironment that stimulates cells to behave as in a specific tissue in vitro or even serve as a functional component part of an artificial organ. Presented below are, by way of example, some approaches that have been analyzed regarding the development of bioengineered in vitro 3D models on nanocellulose containing controllable microstructural and physicochemical features, prepared by the inventors.

3D in vitro model - In vitro embryo production

In order for in vitro animal and human reproduction rates to be improved, in recent years there has been an increasing increase in biotechnologies applied to animal and human reproduction.

The problem is easily established as observed by the rates, for example, of bovine reproduction where only 32.8% of oocytes from females when cultured in vitro reach embryos, and only 33% of these when transferred result in pregnancy.

In vitro embryo production (IVP) involves the stages of collection, maturation, fertilization, and in vitro culture (IVF) with the efficiency of the maturation and fertilization stages in vitro not differing from that obtained in vivo. However, in vitro culture (IVF) is still the stage that presents the lowest efficiency and, on average, only 33% of the oocytes matured in vitro develop to the blastocyst stage. Additionally, blastocysts produced in vitro differ from those produced in vivo, showing lower pregnancy rates and reduced genetic quality.

Although the IVP technique is established, nowadays better conditions of embryonic cell culture are incessantly sought, including the insertion of biologically active molecules and 3D microstructures that can increase the number of embryos produced in relation to the number of oocytes cultured, besides increasing the success in pregnancy percentage in relation to the number of embryos transferred, ensuring the maintenance of genetic quality.

Commonly IVP is performed in plates containing exclusively culture medium, this termed as two-dimensional (2D) culture system. However, such a system offers limitations to embryo development. In the 2D culture system, the embryo adheres to the culture plate, occasion in which the embryo surface in direct contact with the plate suffers limitations regarding the area of exchange between the embryo and the culture medium at the point of adhesion and, consequently, phenotypic alteration that is reflected in the gene expression of embryonic cells. As an attempt to overcome such limitations, three-dimensional (3D) culture systems were developed, where the embryo does not adhere to the plate surface, thus expanding the surface area of exchange between embryo and culture medium and, consequently, with better condition of maintenance of genetic quality of embryos formed.

The biomaterial most used in the 3D embryonic culture model is alginate hydrogel. In some studies, where this biomaterial was used, an improvement in the quality of embryos produced was observed, also providing as an alternative the extension of embryonic culture time. It is reported that 3D culture offers an environment that is physiologically more similar to the maternal uterus when compared to 2D culture. This greater similarity is due to the fact that such a system integrally involves the embryo, allowing its growth even after rupture of the zona pellucida. The disadvantage of the use of alginate hydrogel refers to the low mechanical strength of the biomaterial, which does not allow the transfer of the culture system between the stages involved in IVP. With these limitations in mind, the present invention provides for the development of a nanocellulose 3D matrix that can be altered as to microstructure in terms of porosity and distribution of nanofibers and may or may not contain bioactive molecules adsorbed, absorbed or immobilized on the surface in order to mimic in vitro placental tissue. In addition to the adaptation of the nanocellulose microstructure, the present invention provides the advantage of adsorption, absorption and/or immobilization of bioactive molecules of interest that may or may not be released into the specific media for embryonic cell culture. In this way, the nanocellulose 3D matrix may enable increments to IVP, by providing a larger exchange area between the matrix surface and the cells/embryo and the culture medium, and also by introducing specific molecules in the culture medium that may favor the development of oocytes and/or embryos. An example of a biologically active molecule for the IVP system is melatonin. Melatonin (N-acetylsalicylic-5-methoxytryptamine) is a hormone secreted rhythmically by the pineal gland and is also produced in the ovary, granulosa cells, and oocytes. In mammals, melatonin plays an important role in reproductive activity, especially in species with reproductive seasonality, and is also known to exert an antioxidant action. Melatonin binds to specific receptors and, due to its antioxidant action, plays an important role in protecting ovarian tissues from oxidative stress. During IVP, the manipulation exerted during the procedures involved in performing the technique, as well as the culture conditions themselves, trigger oxidative stress in oocytes and embryos.

Oxidative stress is a biological condition characterized by the imbalance between the production of reactive oxygen species (ROS) and their removal by enzymatic or non-enzymatic systems. ROS promote cell membrane and DNA damage and cause blockage in oocyte maturation and embryo development.

In this context, melatonin possibly constitutes one of the most potent antioxidant substances found in the follicle, directly protecting the oocytes from ROS; however, the effectiveness of such protection seems to be reduced under in vitro culture conditions.

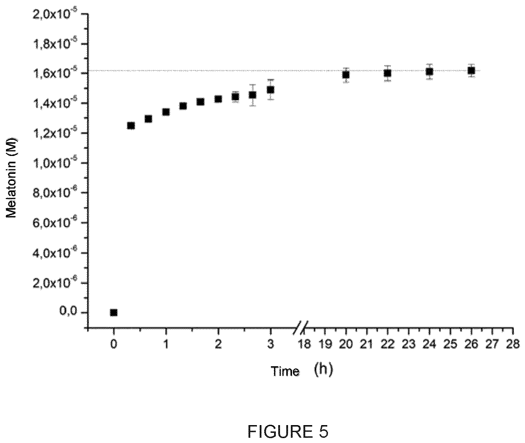

Based on the beneficial effects of melatonin in favoring the removal of ROS, and for its antioxidant and antiapoptotic action, it presents great potential in favoring the development of oocytes and embryos in the in vitro production of the same. Thus, the present invention analyzed the adsorption rate of melatonin on the nanofibers of the nanocellulose 3D matrix containing both surfaces with a dense distribution of nanofibers. FIG. 5 shows the incorporation of melatonin into the nanocellulose 3D matrix for use in in vitro fertilization processes or other steps involved in in vitro embryo production (IVP).

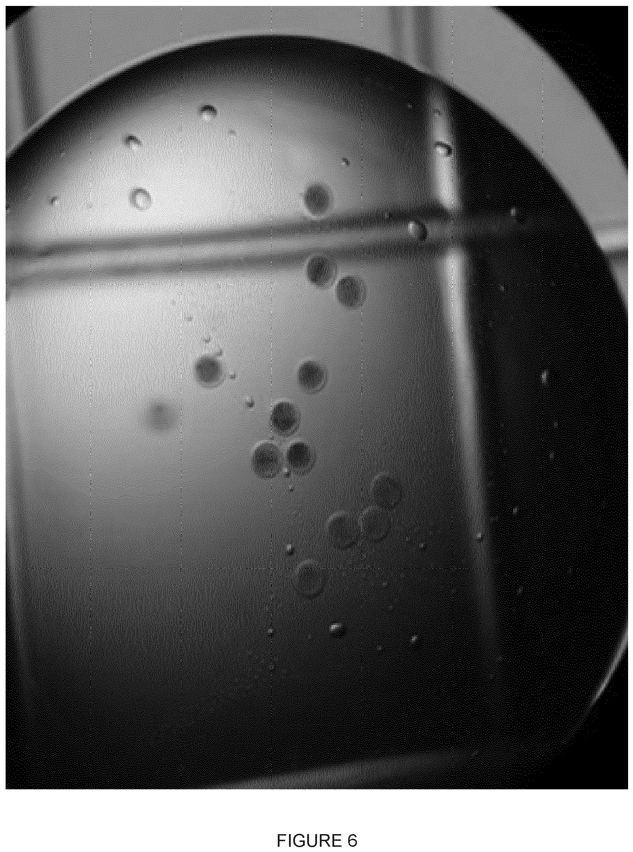

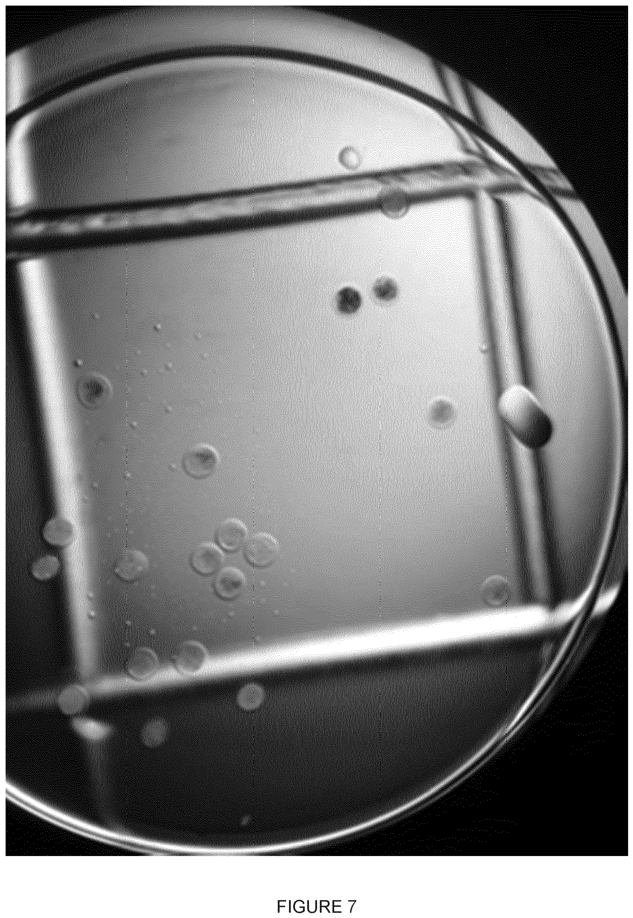

Bovine oocytes were cultured on nanocellulose 3D matrices containing one surface with porous distribution of nanofibers and another surface with dense distribution of nanofibers. FIG. 6 and FIG. 7 show the maturation of oocytes cultured on the 3D nanocellulose arrays.

### In vitro 3D model - Human skin reconstructed in vitro

The present invention features a 3D model of human skin reconstructed on the nanocellulose 3D matrix with microstructure suitable to mimic in vitro the extracellular matrix (ECM) of the skin in vivo. The present invention alternatively claims that the nanocellulose 3D matrix is bioengineered so as to adsorb, absorb, or immobilize biologically active molecules on the nanocellulose 3D matrix. The reconstructed human epidermis on the nanocellulose 3D matrix will be used for efficacy and safety testing of dermocosmetics, general cosmetics, and pharmaceuticals. This invention represents an evolution of biotechnology and tissue engineering techniques and follows the world trend in search of alternative methods to the use of animals, according to a resolution approved by ANVISA (Brazilian Health Regulatory Agency) and aligned with CONCEA principles.

The advantages of in vitro reconstructed human skin on nanocellulose 3D matrix containing bioengineered physicochemical properties in order to increase the uniformity and reproducibility of the tests, as well as the ease of handling of the in vitro models by the manipulator, characterizing a differential property of this product compared to those that have been developed so far. The growth characteristics of human keratinocyte and fibroblast cell lines cultured on the nanocellulose 3D matrix are very similar to those of human skin, thus increasing the uniformity and reproducibility of the tests.

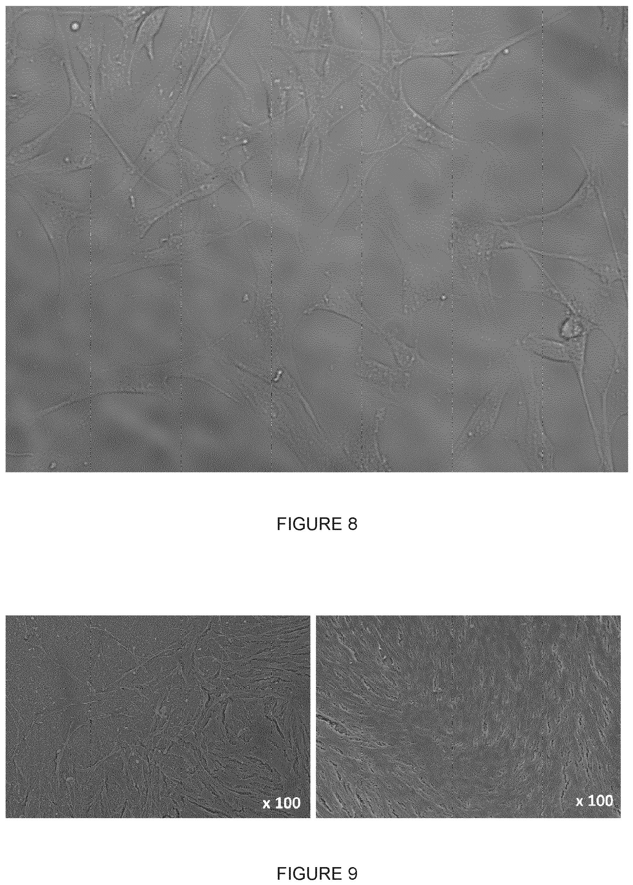

FIG. 8 shows image of reconstructed human skin on the nanocellulose 3D matrix containing a porous distribution of nanofibers on the matrix surface. The image is an enlargement of the self-organization of human fibroblasts, components of the human dermis that adhered, proliferated, and self-organized over the nanofibers of the nanocellulose 3D matrix.

FIG. 9 is a scanning electron microscopy image of human fibroblasts cultured on the nanocellulose 3D matrix on the surface with dense distribution of nanofibers (0.1 to 10%) and demonstrates how the microstructure of nanocellulose 3D matrices influences the behavior of human cells and the in vitro adhesion, proliferation, migration, differentiation, and mimicry of cellular, tissue, and biofunctional biological phenomena. Scanning electron microscopy was used to confirm the adhesion, proliferation of human fibroblasts cultured on the nanocellulose 3D matrix on the surface with dense distribution of nanofibers

To facilitate a better understanding of the present invention, the following examples of certain aspects of some embodiments are provided. In no case should the following examples be read to limit or define the scope of the invention.

### EXAMPLE 1

The bacterium *G. hansenii* inoculated into culture medium containing ammonium chloride and ammonium glutamate as a non-complex nitrogen source. Fermentation occurs in wells of 15.40 mm diameter tissue culture plates at 26°C temperature for 4 days. After purification, the 3D nanocellulose arrays are purified and sterilized. After sterilization, the 3D nanocellulose arrays containing both matrix surfaces with a porous distribution of nanofibers (10 to 99%, porosity) are oxidized in the presence of strong acids that open the polymer chain for immobilization of peptide components of the extracellular matrix. The matrix is subjected to a second sterilization, where the nanofibers of the matrix adsorb specific culture medium for human keratinocytes, during 24 hours of immersion in the solution. After this period, human keratinocytes are seeded on the matrix; they are incubated at 5% CO2 and 37°C for 24 hours in submerged culture. After this period the culture of human keratinocytes adhered to the matrix is transferred to culture at the air-liquid interface for 9 days at 7.5% CO2 and 37°C. After 9 days the humidity of the incubator is reduced and the in vitro reconstructed human epidermis on the nanocellulose 3D matrix is maintained for another three days in culture. After this period the in vitro reconstructed human epidermis is used for efficacy and safety evaluation of cosmetic products, such as in skin irritation and corrosion tests.

### EXAMPLE 2

The bacterium *G. hansenii* inoculated into culture medium containing peptone and yeast extract as a source of complex nitrogen. Fermentation occurs in wells of 6.40 mm diameter tissue culture plates at 26°C temperature for 3 days. After purification the 3D nanocellulose arrays are sterilized. After sterilization the 3D nanocellulose arrays containing one of the surfaces with dense nanofiber distribution (porosity 0.1 to 10%) and the opposite surface containing a porous nanofiber distribution (porosity 10 to 99%) sterilized by moist heat are used as support for adhesion and proliferation of primary human fibroblasts. After immersion of the nanocellulose 3D matrices in DMEM culture medium containing 10% fetal bovine serum, human fibroblasts were cultured and maintained in a humidified atmosphere at 5% CO2 and 37°C for 4 to 7 days. After the culture period, the fibroblasts are fixed with formaldehyde, the matrices are dehydrated with ethanol solutions and subjected to critical point drying for subsequent adhesion analysis by scanning electron microscopy (SEM).

### EXAMPLE 3

The bacterium *G. hansenii* inoculated into culture medium containing peptone and yeast extract as a source of complex nitrogen. Fermentation occurs in wells of 21 mm diameter tissue culture plates at 26°C temperature for 3 days when the arrays are turned over regarding exposure to the air/liquid interface of the fermentation medium; fermentation continues for another 4 days. After production and purification, the 3D nanocellulose arrays are sterilized. After sterilization the 3D nanocellulose arrays containing both sides with dense nanofiber distribution (0.1 to 10% porosity) are sterilized and subsequently submerged in a solution containing 3 × 10⁻⁵ M melatonin for 180 min at 35°C. The melatonin is absorbed into the nanocellulose nanofibers that retain half of the melatonin concentration present in the solution containing the bioactive molecule (melatonin). To perform the in vitro maturation (IVM) procedure, oocytes are cultured on nanocellulose 3D matrix containing the bioactive molecule, melatonin, in its composition (non-covalent modification) containing oocyte maturation base medium associated with 10% fetal bovine serum. The nanocellulose 3D matrix containing both surfaces with a dense distribution of nanofibers where melatonin was absorbed and oocytes seeded is incubated in a culture oven, in humid atmosphere, with 5% CO₂ and 39°C for a period of 24 hours. At the end of this culture the number of blastocysts produced in relation to the number of cultured oocytes is evaluated, and the embryos are morphologically evaluated for quality. Embryo culture is performed under the same conditions as described for the IVM (Example 5). Embryo development will be evaluated at 3, 5, 7 and 9 days post-fecundation. Embryos resulting from in vitro production will be classified according to embryonic development stage into early blastocyst, blastocyst, expanded blastocyst or hatched blastocyst. In addition to the stage of development, embryos are measured for size and classified according to quality into grade I (excellent quality), II (average quality) and III (poor quality) embryos.

### EXAMPLE 4

The bacterium *G. xylinus* inoculated into culture medium containing mannitol as carbon source, micronutrients and peptone and yeast extract as complex nitrogen source. Fermentation occurs in wells of 10.60 mm diameter tissue culture plates at 28°C for 7 days. After purification the nanocellulose 3D matrices are sterilized. After sterilization the 3D nanocellulose arrays containing one of the surfaces with dense nanofiber distribution (porosity 0.1 to 10%) and the opposite surface containing a porous nanofiber distribution (porosity 10 to 99%) is sterilized and further oxidized in the presence of acids for laminin adsorption. After adsorption, the matrices are immersed in cell culture medium for 24 h at 37°C temperature. After this period, fibroblasts of L929 strains are cultured on the matrix in order to evaluate the cytotoxicity of the matrix containing adsorbed laminin. The cytotoxicity was evaluated after 1, 3 and 7 days of cultivation of the cells in contact with the nanocellulose 3D matrix by using colorimetric methods.

### EXAMPLE 5

The bacterium *G. xylinus* inoculated into culture medium containing ammonium sulfate and ammonium nitrate as non-complex nitrogen source and glucose as carbon source. Fermentation occurs in wells of 33.90 mm diameter tissue culture plates at 28°C for 10 days. After purification the 3D nanocellulose arrays are sterilized. After sterilization the 3D nanocellulose arrays containing both surfaces with porous distribution of nanofibers (porosity from 10 to 99%) of nanocellulose are sterilized and subsequently esterified with β-mercaptoethanol. To perform the in vitro maturation (IVM) procedure, oocytes are cultured on the previously esterified nanocellulose 3D matrix containing β-mercaptoethanol immobilized on the nanofibers, and submerged in maturation base medium containing 10% fetal bovine serum. Incubation is performed in a culture oven, in humid atmosphere, with 5% CO₂ and 39°C for a period of 24 hours. At the end of this culture, the number of blastocysts produced in relation to the number of oocytes cultivated is evaluated, and the embryos are morphologically evaluated for quality. Embryo culture is performed under the same conditions as described for the IVM. Embryo development is evaluated at times 3, 5, 7 and 9 post-fecundation. Embryos resulting from in vitro production are classified according to the stage of embryonic development into early blastocyst, blastocyst, expanded blastocyst or hatched blastocyst. In addition to the stage of development, embryos are measured for size and classified according to quality into grade I (excellent quality), II (average quality) and III (poor quality) embryos.

## Claims

1. Nanocellulose 3D matrix for use in the cultivation of animal and human cells in vitro **characterized by** having on one surface a dense distribution of nanofibers containing porosity of 0 to 10%, preferably 0.1 to 2%, and on the opposite surface a porous distribution of nanofibers containing porosity of 10 to 99%, preferably 30 to 60%, with Young's Modulus of 0.05 to 60 MPa, preferably 0.05 to 10 MPa.

2. Nanocellulose 3D matrix for use in the cultivation of animal and human cells in vitro according to claim 1, **characterized in that** it may additionally contain biologically active molecules adsorbed or absorbed into the microstructure of said matrix.

3. Nanocellulose 3D matrix for use in the cultivation of animal and human cells in vitro according to claim 1, **characterized in that** it may additionally contain chemically immobilized, adsorbed or absorbed molecules in the microstructure of said matrix for the purpose of modifying biological activities or physicochemical properties.

4. Nanocellulose 3D matrix for use in in vitro cultivation of animal and human cells according to claim 1, **characterized in that** said specific physical and mechanical properties enable in vitro adhesion, proliferation, migration, differentiation and mimicry of cellular, tissue and biofunctional biological phenomena.

5. Nanocellulose 3D matrix for use in in vitro cultivation of animal and human cells according to claim 1, **characterized in that** said specific physical and mechanical properties enable temporal (4D) analysis of cell behavior and mechanisms related to tissue biofunctionality.

6. Nanocellulose 3D matrix for use in the cultivation of animal and human cells in vitro **characterized by** having on both surfaces a porous distribution of nanofibers containing porosity of 10 to 99%, preferably 30 to 60%, with Young's Modulus of 0.01 to 0.1 MPa, preferably 0.01 to 0.076 MPa.

7. Nanocellulose 3D matrix for use in the cultivation of animal and human cells in vitro according to claim 6, **characterized in that** it may additionally contain biologically active molecules adsorbed or absorbed into the microstructure of said matrix.

8. Nanocellulose 3D matrix for use in the cultivation of animal and human cells in vitro according to claim 6, **characterized in that** it may additionally contain chemically immobilized, adsorbed or absorbed molecules in the microstructure of said matrix for the purpose of modifying biological activities or physicochemical properties.

9. Nanocellulose 3D matrix for use in the cultivation of animal and human cells in vitro according to claim 6, **characterized in that** said specific physical and mechanical properties enable in vitro adhesion, proliferation, migration, differentiation and mimicry of cellular, tissue and biofunctional biological phenomena.

10. Nanocellulose 3D matrix for use in in vitro cultivation of animal and human cells according to claim 6, **characterized in that** said specific physical and mechanical properties enable temporal (4D) analysis of cell behavior and mechanisms related to tissue biofunctionality.

11. Method for manufacturing nanocellulose 3D matrix by modulating the composition of the culture medium as to carbon, nitrogen and micronutrient source, **characterized in that** it comprises fermentation step in culture medium having a complex nitrogen source for 1 to 10 days at temperature from 25°C to 30°C.

12. Method for manufacturing nanocellulose 3D matrix by modulating the composition of the culture medium as to carbon, nitrogen and micronutrient source according to claim 11, **characterized in that** said method produces matrix having dense nanofiber distribution on one surface and porous on the opposite surface.

13. Method for manufacturing nanocellulose 3D matrix by modulating the composition of the culture medium as to carbon, nitrogen and micronutrient source according to claim 11, **characterized in that** said manufacturing method may additionally contain the step of adsorbing or absorbing biologically active molecules into the microstructure of said matrix.

14. Method for manufacturing nanocellulose 3D matrix by modulating the composition of the culture medium as to carbon, nitrogen and micronutrient source according to claim 11, **characterized in that** said manufacturing method may additionally contain the step of immobilizing or chemically adsorbing molecules onto the microstructure of said matrix for the purpose of modifying biological activities or physicochemical properties.

15. Method for manufacturing nanocellulose 3D matrix by modulating the composition of the culture medium as to carbon, nitrogen and micronutrient source according to claim 11, **characterized in that** said specific nanofiber distribution enables in vitro adhesion, proliferation, migration, differentiation and mimicry of cellular, tissue and biofunctional biological phenomena.

16. Method for manufacturing nanocellulose 3D matrix by modulating the composition of the culture medium as to carbon, nitrogen and micronutrient source according to claim 11, **characterized in that** said specific nanofiber distribution enables temporal (4D) analysis of cell behavior and mechanisms related to tissue biofunctionality.

17. Method for manufacturing nanocellulose 3D matrix by modulating the composition of the culture medium regarding carbon, nitrogen and micronutrient source, **characterized in that** it comprises fermentation step in culture medium having a non-complex nitrogen source for 3 to 10 days at temperature from 25°C to 30°C.

18. Nanocellulose 3D matrix manufactured by modulating the composition of the culture medium as to the source of carbon, nitrogen and micronutrients, **characterized in that** said matrix is obtained through a manufacturing method comprising a fermentation step in a culture medium having a complex nitrogen source for 1 to 10 days, at a temperature of 25°C to 30°C.

19. Nanocellulose 3D matrix manufactured by modulating the composition of the culture medium as to the source of carbon, nitrogen and micronutrients, **characterized in that** said matrix is obtained through a manufacturing method comprising a fermentation step in a culture medium having a complex nitrogen source for 3 to 5 days, followed by a new fermentation step with the opposite face exposed for 3 to 5 days, at a temperature of 25°C to 30°C.

20. Nanocellulose 3D matrix manufactured by modulating the composition of the culture medium as to the source of carbon, nitrogen and micronutrients, **characterized in that** said matrix is obtained through a manufacturing method that comprises a fermentation step in a culture medium that has a non-complex nitrogen source for 3 to 10 days, at a temperature of 25°C to 30°C.

21. Nanocellulose 3D matrix manufactured by modulating the composition of the culture medium as to carbon, nitrogen and micronutrient source according to claim 20, **characterized in that** said method produces matrix with porous nanofiber distribution on both surfaces.

22. Nanocellulose 3D matrix manufactured by modulating the composition of the culture medium as to carbon, nitrogen and micronutrient source according to claim 21, **characterized in that** said matrix produced by said method exhibits porous nanofiber distribution on both surfaces with porosity ranging from 10 to 99%, preferably 30 to 60%.

23. Nanocellulose 3D matrix manufactured by modulating the composition of the culture medium as to carbon, nitrogen and micronutrient source according to claim 20, **characterized in that** said manufacturing method may additionally contain the step of adsorption or absorption of biologically active molecules in the microstructure of said matrix.

24. Nanocellulose 3D matrix manufactured by modulating the composition of the culture medium as to carbon, nitrogen and micronutrient source according to claim 20, **characterized in that** said manufacturing method may additionally comprise the step of immobilization or chemical adsorption of molecules on the microstructure of said matrix for the purpose of modifying biological activities or physicochemical properties.

25. Nanocellulose 3D matrix manufactured by modulating the composition of the culture medium as to carbon, nitrogen and micronutrient source according to claim 21, **characterized in that** said specific nanofiber distribution enables in vitro adhesion, proliferation, migration, differentiation and mimicry of cellular, tissue and biofunctional biological phenomena.

26. Nanocellulose 3D matrix manufactured by modulating the composition of the culture medium as to carbon, nitrogen and micronutrient source according to claim 21, **characterized in that** said specific nanofiber distribution enables temporal (4D) analysis of cell behavior and mechanisms related to tissue biofunctionality.

27. Use of nanocellulose 3D matrix having dense nanofiber distribution on one surface and porous on the opposite surface, **characterized in that** it is for use in 3D cultivation of animal and human cells in vitro.

28. Use of nanocellulose 3D matrix with dense nanofiber distribution on one of the surfaces and porous on the opposite surface, **characterized in that** it is for use in 3D culture of animal and human embryonic cells in in vitro fertilization processes.

29. Use of nanocellulose 3D matrix with dense nanofiber distribution on one of the surfaces and porous on the opposite surface, **characterized in that** it is for use as 3D support for in vitro reconstructed human skin growth from human and animal cells for use in efficacy and safety testing of dermocosmetics.

30. Use of nanocellulose 3D matrix with porous nanofiber distribution on both surfaces, **characterized in that** it is for use in 3D culture of animal and human cells in vitro.

31. Use of nanocellulose 3D matrix with porous nanofiber distribution on both surfaces, **characterized in that** it is for use in 3D culture of animal and human embryonic cells in in vitro fertilization processes.

32. Use of nanocellulose 3D matrix with porous nanofiber distribution on both surfaces, **characterized in that** it is for use as 3D support for in vitro reconstructed human skin growth from human and animal cells for use in efficacy and safety testing of dermocosmetics.
